Europäisches Patentamt

European Patent Office

Office européen des brevets

(19)

(11) Veröffentlichungsnummer: **0 147 710**
**B1**

(12) **EUROPÄISCHE PATENTSCHRIFT**

(45) Veröffentlichungstag der Patentschrift:
08.03.89

(21) Anmeldenummer: 84115063.4

(22) Anmeldetag: 10.12.84

(51) Int. Cl.⁴: **A 61 N 1/05,** C 23 C 14/34,
G 01 N 27/07, G 01 N 27/30

(54) Verfahren zum Herstellen einer implantierbaren Elektrode.

(30) Priorität: 20.12.83 DE 3345990

(43) Veröffentlichungstag der Anmeldung:
10.07.85 Patentblatt 85/28

(45) Bekanntmachung des Hinweises auf die Patenterteilung:
08.03.89 Patentblatt 89/10

(84) Benannte Vertragsstaaten:
DE FR GB IT NL

(56) Entgegenhaltungen:
DE-A-2 604 165
DE-A-3 116 040
DE-B-2 613 052
DE-B-2 613 072

CARBON, Vol. 14, 1976, Pergamon Press, Oxford-
New York-Paris-Frankfurt Z. MARINKOVIC, R. ROY
"Preparation and Properties of sputtered "glassy"
carbon films" Seiten 329-331

(73) Patentinhaber: Siemens Aktiengesellschaft Berlin
und München, Wittelsbacherplatz 2, D-8000
München 2 (DE)

(72) Erfinder: Edeling, Martin, Dr., Weiherstrasse 17,
D-8520 Erlangen (DE)
Erfinder: Freller, Helmut, Steinbergstrasse 34a,
D-8505 Röthenbach/P (DE)
Erfinder: Mund, Konrad, Dr., Langenbrucker Weg
10, D-8525 Uttenreuth (DE)
Erfinder: Schack, Peter, Schnieglinger Strasse 33,
D-8500 Nürnberg (DE)

vorsehen, die mit einer dünnen, beispielsweise einigen µm dicken, Platinschicht versehen ist.

Die Glaskohlenstoffschicht sputtert man von dem Glaskohlenstoff-Target beispielsweise in einer Argonatmosphäre bei einem Druck von beispielsweise 4 bis 8 · 10⁻² mbar, vorzugsweise etwa 5 bis 7 · 10⁻² mbar, insbesondere etwa 6 · 10⁻² mbar, und einer Spannung von beispielsweise 1,6 bis 2,4 kV, vorzugsweise 1,8 bis 2,2 kV, insbesondere etwa 2,1 kV. Die Leistung kann beispielsweise 0,5 bis 1,5 kW, vorzugsweise 0,8 bis 1,2 kW, insbesondere etwa 1 kW, und die Abscheidungsrate beispielsweise etwa 15 nm pro Minute betragen.

Die auf die Elektrode aufgesputterte Glaskohlenstoffschicht weist eine Dicke zwischen 1 und 20 µm auf, vorzugsweise eine Dicke von ca. 4 bis 5 µm, und erreicht etwa eine Dichte von 0,7 g/cm³ Somit erhält man keine dichte, sondern eine mikroporöse Oberfläche der Elektrode, d.h. eine Oberfläche mit Poren, deren Durchmesser kleiner als etwa 0,002 µm ist, und deren Dichte etwa die Hälfte von dichtem Glaskohlenstoff erreicht, wobei aber der Elektrodenoberfläche die glatte Oberfläche makroskopisch erhalten bleibt. Entgegen der Erkenntnis der Praxis, nämlich der Proportionalität zwischen Argonatmosphärendruck und Abscheidungsrate und der proportionalität zwischen Leistung und Dichte, erhält man hier bei einer verhältnismäßig hohen Leistung von beispielsweise etwa 1 kW nur eine verhältnismäßig geringe Dichte von beispielsweise etwa 0,7 g/cm³. Außerdem haftet diese aufgesputterte Glaskohlenstoffschicht gut auf der Elektrode, beispielsweise sehr gut auf einer Platinelektrode.

Ferner erreicht eine Platinelektrode mit einer aufgesputterten porösen Glaskohlenstoffschicht bei einer Frequenz von etwa 1 Hz beispielsweise eine hohe elektrochemische Doppelschichtkapazität von etwa 50 mF/cm². Somit erhält man eine implantierbare Elektrode, deren Polarisationsverluste sehr niedrig sind, die an der Grenzfläche zwischen Elektrode und Gewebe auftreten und die nicht zur Erhöhung der Feldstärke im angrenzenden reizbaren Gewebe beitragen. Außerdem ist bei dieser implantierbaren Elektrode sowohl eine geringe Abkapselung durch Bindegewebsbildung als auch ein geringer Energieverbrauch und damit verbunden ein gutes Dauerbetriebsverhalten gewährleistet, weil die Stromdichte der Reizschwelle während der Dauer der Implantation sich nicht erhöht.

Diese Vorteile, insbesondere die hohe elektrochemische Doppelschichtkapazität, erhält man, ohne dabei den Glaskohlenstoff zusätzlich zu aktivieren, und außerdem behält man bei einer Platinelektrode den Vorteil der guten Kontaktierung. Da Platin zu den metallischen Materialien gehört, kann man die Elektrode auf einfache Weise herstellen und gestalten.

In einer vorteilhaften Ausführungsform der implantierbaren Elektrode kann man die Elektrode, die beispielsweise aus Platin besteht, wenigstens teilweise mit Glaskohlenstoff besputtern. Somit erreicht man, daß die Strombelastung wegen der niedrigen Impedanz sich auf diesen besputterten Bereich konzentriert. Durch die Variationen dieser Flächen auf der Oberfläche des Elektrodenkopfes erhält man eine wesentliche Verbesserung hinsichtlich des Energieaufwandes und der Reizschwelle.

In einer weiteren vorteilhaften Ausführungsform sind Platindrähte für Mikroelektroden oder Mehrfachelektroden für die Cochlea des künstlichen Ohrs mit Glaskohlenstoff besputtert. Bei dieser Anwendung ist eine große flächenbezogene Doppelschichtkapazität besonders vorteilhaft, weil bei der Nervenreizung hohe Stromdichten, beispielsweise 0,5 A/cm², wegen der Reizschwelle gefordert werden und somit hohe Polarisationswerte erreicht werden können.

**Patentansprüche**

1. Verfahren zum Herstellen einer implantierbaren Elektrode, insbesondere Reizelektrode, dadurch gekennzeichnet, daß von einem Glaskohlenstoff-Target Glaskohlenstoff auf wenigstens einen Teil der Oberfläche der Elektrode aufgesputtert wird

2. Verfahren nach Anspruch 1, dadurch gekennzeichnet, daß in einer Argonatmosphäre gesputtert wird.

3. Verfahren nach Anspruch 1 oder 2, dadurch gekennzeichnet, daß bei einem Druck von 4 bis 8 · 10⁻² mbar gesputtert wird.

4. Verfahren nach einem der Ansprüche 1 bis 3, dadurch gekennzeichnet, daß bei einer Spannung von 1,6 bis 2,4 kV gesputtert wird.

5. Verfahren nach einem oder mehreren der Ansprüche 1 bis 4, dadurch gekennzeichnet, daß mit einer Leistung von 0,5 bis 1,5 kW gesputtert wird.

6. Implantierbare Elektrode nach einem oder mehreren der Ansprüche 1 bis 5, dadurch gekennzeichnet, daß als Material der Elektrode Platin vorgesehen ist.

7. Implantierbare Elektrode nach einem oder mehreren der Ansprüche 1 bis 5, dadurch gekennzeichnet, daß als Material der Elektrode Platin-Iridium vorgesehen ist.

8. Implantierbare Elektrode nach einem oder mehreren der Ansprüche 1 bis 5, dadurch gekennzeichnet, daß als Material der Elektrode Glaskohlenstoff vorgesehen ist.

9. Implantierbare Elektrode nach einem oder mehreren der Ansprüche 1 bis 5, dadurch gekennzeichnet, daß die Elektrode aus einer Legierung besteht, die Kobalt, Chrom, Eisen, Nickel, Molybdän und Mangan enthält, die mit einer Platinschicht versehen ist.

## Claims

1. A method of producing an implantable electrode, in particular a stimulating electrode, characterised in that vitreous carbon is sputtered on to at least part of the electrode surface from a vitreous carbon target.

2. A method according to claim 1, characterised in that the sputtering is performed in an argon atmosphere.

3. A method according to claim 1 or claim 2, characterised in that the sputtering is performed under a pressure of 4 to 8 · $10^{-2}$ mbar.

4. A method according to any one of claims 1 to 3, characterised in that the sputtering is performed at a voltage of 1.6 to 2.4 kV.

5. A method according to any one or more of claims 1 to 4, characterised in that the sputtering is performed at a power of 0.5 to 1.5 kV.

6. An implantable electrode according to any one or more of claims 1 to 5, characterised in that the material of the electrode is platinum.

7. An implantable electrode according to any one or more of claims 1 to 5, characterised in that the material of the electrode is platinum-iridium.

8. An implantable electrode according to any one or more of claims 1 to 5, characterised in that the material of the electrode is vitreous carbon.

9. An implantable electrode according to any one or more of claims 1 to 5, characterised in that the electrode consists of an alloy containing cobalt, chromium, iron, nickel, molybdenum and manganese that is coated with a layer of platinum.

## Revendications

1. Procédé pour fabriquer une électrode implantable, notamment une électrode de simulation, caractérisé par le fait qu'on pulvérise cathodiquement du carbone vitreux, à partir d'une cible en carbone vitreux, sur au moins une partie de la surface de l'électrode.

2. Procédé suivant la revendication 1, caractérisé par le fait qu'on réalise la pulvérisation cathodique dans une atmosphère d'argon.

3. Procédé suivant la revendication 1 ou 2, caractérisé par le fait qu'on réalise la pulvérisation cathodique sous une pression comprise entre 4 et 8 · $10^{-2}$ m.bars.

4. Procédé suivant l'une des revendications 1 à 3, caractérisé par le fait qu'on réalise la pulvérisation cathodique avec une tension comprise entre 1,6 et 2,4 kV.

5. Procédé suivant l'une ou plusieurs des revendications 1 à 4, caractérisé par le fait qu'on réalise la pulvérisation cathodique avec une puissance comprise entre 0,5 et 1,5 kW.

6. Electrode implantable suivant une ou plusieurs des revendications 1 à 5, caractérisé par le fait qu'on prévoit du platine comme matériau de l'électrode.

7. Electrode implantable suivant une ou plusieurs des revendications 1 à 5, caractérisée par le fait qu'on prévoit du platine-iridium comme matériau de l'électrode.

8. Electrode implantable suivant une ou plusieurs des revendications 1 à 5, caractérisée par le fait qu'on prévoit du carbone vitreux comme matériau de l'électrode.

9. Electrode implantable suivant une ou plusieurs des revendications 1 à 5, caractérisée par le fait que l'électrode est constituée par un alliage qui contient du cobalt, du chrome, du fer, du nickel, du molybdène et du manganèse et est recouverte d'une couche de platine.